## Europäisches Patentamt
## European Patent Office
## Office européen des brevets

(19)

(11) Veröffentlichungsnummer: **0 050 868**
**B1**

(12) **EUROPÄISCHE PATENTSCHRIFT**

(45) Veröffentlichungstag der Patentschrift:
**03.10.84**

(21) Anmeldenummer: **81108897.0**

(22) Anmeldetag: **25.10.81**

(51) Int. Cl.³: **C 07 C 85/20,** C 07 C 87/06,
C 07 C 67/20, C 07 C 69/06

(54) **Verfahren zur Gewinnung von N-tert-Alkylaminen und Ameisensäureestern.**

(30) Priorität: **27.10.80 DE 3040406**

(43) Veröffentlichungstag der Anmeldung:
**05.05.82 Patentblatt 82/18**

(45) Bekanntmachung des Hinweises auf die Patenterteilung:
**03.10.84 Patentblatt 84/40**

(84) Benannte Vertragsstaaten:
**AT BE CH DE FR GB LI NL**

(56) Entgegenhaltungen:
**DE - B - 1 051 862**
**DE - B - 1 059 468**
**DE - C - 523 189**

(73) Patentinhaber: **Degussa Aktiengesellschaft,
Weissfrauenstrasse 9, D-6000 Frankfurt am Main 1 (DE)**

(72) Erfinder: **Giesselmann, Günther, Dr. Dipl.-Chem.,
Grafenbrucher Weg 17, D-6056 Heusenstamm (DE)**
Erfinder: **Günther, Kurt, Dr. Dipl.-Chem.,
Goethestrasse 4, D-6455 Erlensee (DE)**
Erfinder: **Schalke, Peter, Dr. Dipl.-Chem., Südring 74,
D-6458 Rodenbach (DE)**

**Beschreibung**

Die Erfindung betrifft ein Verfahren zur Gewinnung von N-tert.-Alkylaminen und Estern der Ameisensäure aus den bei der Hydrolyse von N-tert.-Alkylformamiden anfallenden Umsetzungsgemischen.

Bei der Hydrolyse der N-tert.-Alkylformamide entstehen in äquivalenten Mengen die entsprechenden N-tert.-Alkylamine und Ameisensäure. Diese Substanzen liegen in Abhängigkeit von den pH-Werten in den Umsetzungsgemischen gegebenenfalls als Salze vor. Die Ameisensäure ist wie das N-tert.-Alkylamin so wertvoll, dass es notwendig ist, beide Substanzen aus den Umsetzungsgemischen zu gewinnen.

Es ist bekannt, N-tert.-Alkylformamide zu den entsprechenden N-tert.-Alkylaminen zu hydrolysieren und die Hydrolyse mittels Alkalihydroxid (US-PS 2 773 097) oder mittels starker Säure (DE-AS 1 051 682) durchzuführen. Die Amine werden aus dem Umsetzungsgemisch durch Destillation abgetrennt. Über die Gewinnung der Ameisensäure wird nichts ausgesagt.

Es ist auch bekannt, N-tert.-Alkylformamide wahlweise mittels starker Basen oder starker Säuren zu hydrolysieren und im Falle der Hydrolyse mittels Chlorwasserstoffsäure aus dem Umsetzungsgemisch zunächst die Ameisensäure und dann nach Zugabe von Basen das Amin abzudestillieren (DE-PS 870 856). Nachteilig ist bei diesem Verfahren, dass die Ameisensäure nur unvollständig gewonnen werden kann und in Form verdünnter wässriger Lösungen anfällt, die zudem durch Chlorwasserstoff verunreinigt sind. Überdies ist die Ausbeute an dem Amin mässig.

Es ist schliesslich bekannt, tert.-Butylformamid mittels Natriumhydroxid zu hydrolysieren, zunächst aus dem Umsetzungsgemisch das tert.-Butylamin abzudestillieren, dann in dem restlichen Umsetzungsgemisch die Ameisensäure mittels Schwefelsäure freizusetzen und mit Methanol zu verestern und schliesslich den Ameisensäuremethylester abzudestillieren (US-PS 4 131 642). Bei diesem Verfahren ist die Ausbeute sowohl an dem tert.-Butylamin als auch an dem Ameisensäureester unbefriedigend.

Es ist nun ein Verfahren zur Gewinnung von N-tert.-Alkylaminen und Estern der Ameisensäure durch Destillation aus den bei der Hydrolyse von N-tert.-Alkylformamiden anfallenden Umsetzungsgemischen gefunden worden, das dadurch gekennzeichnet ist, dass die Hydrolyse mittels Halogenwasserstoff oder Schwefelsäure oder deren Mischungen in Gegenwart von Wasser und niederen Alkanolen durchgeführt, die Ester der Ameisensäure abgetrennt, die verbleibenden Gemische mit stark alkalisch wirkenden Substanzen versetzt und dann die Amine abgetrennt werden. Bei diesem Verfahren fallen sowohl die Ester der Ameisensäure als auch die Amine mit hervorragender Ausbeute an.

Das Verfahren eignet sich insbesondere für die Gewinnung des tert.-Butylamins und tert.-Amylamins sowie der Ester der Ameisensäure mit Äthanol, Propanol-(2) oder vorzugsweise Methanol aus den bei der Hydrolyse von tert.-Butylformamid oder tert.-Amylformamid in Gegenwart von Äthanol, Propanol-(2) oder vorzugsweise Methanol anfallenden Umsetzungsgemischen. Zweckmässigerweise wird als Halogenwasserstoff Bromwasserstoff oder vorzugsweise Chlorwasserstoff eingesetzt und als alkalisch wirkende Substanz ein Alkali- oder Erdalkalihydroxid, vorzugsweise Natriumhydroxid, Kaliumhydroxid oder Calciumhydroxid, verwendet.

Die Mengenverhältnisse können in weiten Bereichen gewählt werden. Im allgemeinen ist es zweckmässig je Mol des N-tert.-Alkylformamids wenigstens etwa 1 Mol des Alkanols, 1 Mol des Halogenwasserstoffs und 1 Mol Wasser einzusetzen. Vorzugsweise werden je Mol Formamid, 1,05 bis 2,0 Mol, insbesondere 1,05 bis 1,50 Mol, Alkanol sowie Halogenwasserstoff und 2,0 bis 6,0 Mol Wasser genommen. Beispielsweise ist es im Falle der Verwendung von Chlorwasserstoff vorteilhaft, diesen und das Wasser in Form einer konzentrierten wässrigen Chlorwasserstoffsäure einzubringen. Der Halogenwasserstoff kann teilweise oder ganz durch gleichmolare Mengen Schwefelsäure ersetzt werden. Die alkalisch wirkende Substanz wird zweckmässigerweise in solchen Mengen eingesetzt, dass die Mischung einen pH-Wert von wenigstens etwa 12, vorzugsweise einen pH-Wert von mehr als 13, aufweist.

Die Temperaturen, bei denen das erfindungsgemässe Verfahren durchgeführt wird, richten sich in gewissem Umfang nach der Art der Substanzen. Im allgemeinen kommen Temperaturen etwa zwischen 20°C und dem Siedepunkt des Umsetzungsgemisches in Frage. Wenngleich der Druck beliebig gewählt werden kann, ist es zweckmässig, bei Normaldruck oder von diesem nicht wesentlich abweichendem Druck zu arbeiten.

Die Ester der Ameisensäure und die Amine werden aus dem Umsetzungsgemisch durch Destillation abgetrennt. Hierzu ist es vorteilhaft, mit der Hydrolyse bei Temperaturen von etwa 20 bis 50°C zu beginnen und dann die Temperatur so einzustellen, dass der Ester der Ameisensäure und, nach Zugabe der alkalisch wirkenden Substanz, das Amin aus dem Umsetzungsgemisch abdestilliert.

*Beispiel 1*

Es wurde eine Mischung aus 101 g (1,0 Mol) tert.-Butylformamid und 42 g (1,3 Mol) Methanol vorgelegt und in diese Mischung wurden 100 ml konzentrierte wässrige Chlorwasserstoffsäure (entsprechend 1,3 Mol Chlorwasserstoff und 4,0 Mol Wasser) eingetragen. Die Mischung erwärmte sich hierbei auf Siedetemperatur und wurde weiter auf dieser Temperatur gehalten, bis der Ameisensäuremethylester abdestilliert war. Gewonnen wurden 59 g Ester; dessen Siedepunkt war 32°C. Die Reinheit war 99%; die Ausbeute an reinem Ester, bezogen auf das eingesetzte tert.-Butylformamid, betrug 98%. Aus dem verbliebenen Umsetzungsgemisch wurde bei 60 bis 100°C das nicht umgesetzte Methanol abgetrieben. Dann wurde das Umsetzungsgemisch auf 45°C abgekühlt und durch Zugabe einer Lösung von 55 g Natriumhydroxid in 120 ml Wasser auf den pH-Wert 14 gebracht. Schliesslich wurde das tert.-Butylamin abdestilliert. Die Reinheit des Amins war 99%. Es wurden 71 g gewonnen, entsprechend einer Ausbeute an reinem Amin von 96%, bezogen auf das eingesetzte tert.-Butylformamid.

*Beispiel 2*

Es wurde wie nach Beispiel 1 verfahren, jedoch wurden statt Methanol 130 ml (1,7 Mol) Propanol-(2) eingesetzt. Gewonnen wurden 83 g Ameisensäurereester des Propanols-(2) mit dem Siedepunkt 67 bis 71°C. Die Reinheit war 95%; die Ausbeute an reinem Ester, bezogen auf das eingesetzte tert.-Butylformamid, betrug 89%. Ausserdem fielen 68 g tert.-Butylamin mit dem Siedepunkt 45 bis 47°C an. Die Reinheit war 95%; die Ausbeute an reinem Amin, bezogen auf das eingesetzte Formamid, betrug 89%.

*Beispiel 3*

Es wurde wie nach Beispiel 1 verfahren, jedoch wurden statt tert.-Butylformamid 115 g (1,0 Mol) tert.-Amylformamid eingesetzt. Gewonnen wurden 57 g Ameisensäuremethylester. Die Reinheit war 94%; die Ausbeute an reinem Ester, bezogen auf das eingesetzte tert.-Amylformamid, betrug 95%. Ausserdem fielen 86 g tert.-Amylamin an. Die Reinheit war 92%; die Ausbeute an reinem Amin, bezogen auf das eingesetzte Formamid, betrug 90%.

*Beispiel 4*

In 120,8 g 89,3%iger Schwefelsäure (1,1 Mol) wurden nacheinander 57,7 g (1,8 Mol) Methanol und 101,0 g (1,0 Mol) tert.-Butylformamid eingetragen. Die Mischung wurde eine Stunde lang unter Rückfluss auf Siedetemperatur gehalten. Dann wurde der Ameisensäuremethylester und nach Zugabe von 95 g Wasser das überschüssige Methanol abgetrieben. Schliesslich wurde nach Zugabe von 196 g einer 45%igen wässrigen Natriumhydroxydlösung das tert.-Butylamin abdestilliert. Gewonnen wurden 61 g Ester (Reinheit 97%), entsprechend 99% Ausbeute, und 72 g Amin (Reinheit 99%), entsprechend 97% Ausbeute, bezogen auf das eingesetzte Formamid.

**Patentanspruch**

Verfahren zur Gewinnung von N-tert.-Alkylaminen und Estern der Ameisensäure durch Destillation aus den bei der Hydrolyse von N-tert.-Alkylformamiden anfallenden Umsetzungsgemischen, dadurch gekennzeichnet, dass die Hydrolyse mittels Halogenwasserstoff oder Schwefelsäure oder deren Mischungen in Gegenwart von Wasser und niederen Alkanolen durchgeführt wird, die Ester der Ameisensäure abdestilliert, die verbleibenden Gemische mit stark alkalisch wirkenden Substanzen versetzt und dann die Amine abdestilliert werden.

**Claim**

A process for the recovery of N-tert.-alkyl amines and esters of formic acid by distillation from the reaction mixtures which are produced during the hydrolysis of N-tert.-alkyl formamides, characterised in that hydrolysis is carried out with hydrogen halide or sulphuric acid or the mixtures thereof in the presence of water and lower alkanols, the esters of formic acid are distilled off, the remaining mixtures are mixed with strongly alkaline-acting substances and the amines are subsequently distilled off.

**Revendication**

Procédé d'obtention de N-tert.-alcoylamines et d'esters de l'acide formique, par distillation du mélange réactionnel obtenu lors de l'hydrolyse de N-tert.-alcoylformamides, procédé caractérisé en ce que l'hydrolyse est effectuée au moyen d'un hydracide ou d'acide sulfurique, ou de leurs mélanges, en présence d'eau et d'alcanols inférieurs, qu'on reprend l'ester formique par distillation, ajoute au mélange restant une substance à action fortement alcaline, et ensuite, reprend l'amine par distillation.